# EUROPÄISCHE PATENTSCHRIFT

(11) **EP 0 486 775 B1**
(45) Veröffentlichungstag und Bekanntmachung des Hinweises auf die Patenterteilung: **23.11.1994**
(21) Anmeldenummer: 91114440.0
(22) Anmeldetag: 28.08.1991
(51) Int. Cl.: A61K 6/083, A61K 6/00

(54) **Polymerisierbares Dentalmaterial**
Polymerisable dental material
Matériau dentaire polymérisable

(30) Priorität: 17.11.1990 DE 4036675; 02.04.1991 DE 4110612
(43) Veröffentlichungstag der Anmeldung: 27.05.1992
(73) Patentinhaber: HERAEUS KULZER GMBH, 63450 Hanau (DE)
(72) Erfinder: Eppinger, Regina, W-6290 Weilburg (DE); Eppinger, Bernhard, W-6290 Weilburg (DE); Heindl, Detlef, W-6294 Weinbach (DE); Kohler, Peter Jochen, W-6350 Bad Nauheim (DE); Fritze, Joachim, Dr., W-6382 Friedrichsdorf (DE)
(74) Vertreter: Kühn, Hans-Christian

(56) Entgegenhaltungen:
- EP-A- 0 053 442
- EP-A- 0 116 121
- EP-A- 0 156 105
- EP-A- 0 176 777
- EP-A- 0 382 033
- DE-A- 4 002 726

## Beschreibung

Die Erfindung betrifft ein polymerisierbares Dentalmaterial, enthaltend monomere Dimethacrylate, ein anorganisches Füllstoff-Gemisch aus Bariumaluminiumsilicat-Glas und mikrofeinem Siliciumdioxid und ein α-Diketon/Amin-System für die Photopolymerisation.

Das erfindungsgemäße Dentalmaterial stellt ein Zahnfüllungsmaterial, ein Material zur Herstellung von Inlays und einen dentalen Befestigungszement dar.

Polymerisierbare Zahnfüllungsmaterialien sind seit vielen Jahren bekannt. Die ersten dieser Materialien bestanden aus Mischungen aus monomerem und polymerem Methylmethacrylat, die durch Zusatz eines Katalysators bzw. eines Systems aus Katalysator und Beschleuniger innerhalb weniger Minuten unter den im Munde herrschenden Temperaturbedingungen aushärteten.

Eine Verbesserung der mechanischen Eigenschaften dieser Füllungsmaterialien wurde durch den Zusatz von feinteiligen Füllstoffen wie Quarz oder Aluminiumsilicaten, eine Verbesserung der ästhetischen Wirkung durch die Entwicklung neuer Katalysator-Systeme, die keine Verfärbung mehr verursachen, und eine Verringerung der Polymerisationsschrumpfung durch die Verwendung von Methacrylsäureestern höherer Alkohole neben oder anstelle von Methylmethacrylat erreicht.

Das erste dieser neuen Materialien wurde von Rafael L. Bowen entwickelt und ist in der US-Patentschrift 3 066 112 beschrieben. Es enthält als monomeres Bindemittel im wesentlichen ein durch Reaktion eines Bis-Phenols mit Glycidylacrylat bzw. -methacrylat dargestelltes Diacrylat bzw. Dimethacrylat und als anorganischen Füllstoff feinteiliges Siliciumdioxid, vorzugsweise in silanisierter Form. Das von Bowen gefundene Bis-[4-(2-hydroxy-3-methacryloyloxypropoxy)-phenyl]-dimethylmethan, auch als Bis-GMA oder Bowen-Monomer bezeichnet, ist auch heute noch in den meisten der im Handel befindlichen Dentalmaterialien enthalten.

Ein Beispiel für ein weiteres Composite - ein Dentalmaterial, das neben organischen Monomeren einen feinteiligen anorganischen Füllstoff enthält - wird in der US-Patentschrift 3 539 533 beschrieben. Das polymerisierbare Bindemittel ist dabei eine Mischung aus Bis-GMA, Bisphenol A-dimethacrylat, verdünnendem Monomer, besonders Triäthylenglykoldimethacrylat, und gegebenenfalls Methacrylsäure in geringer Menge, die zusammen mit etwa 65-75 Gewichts-% des anorganischen Füllstoffes, zum Beispiel aus Siliciumdioxid, Glas, Aluminiumoxid oder Quarz, verwendet wird. Der anorganische Füllstoff kann eine Teilchengröße von etwa 2 - 85 Mikrometer besitzen; zur Verbesserung des Verbundes Kunststoff/Füllstoff wird er mit einem Silan, wie zum Beispiel 3-Methacryloyloxypropyltrimethoxysilan, vorbehandelt.

Aus der deutschen Patentschrift 24 03 211 ist ein Werkstoff für Dentalzwecke (Füllungsmaterialien für Kavitäten, Materialien für Befestigungszemente, Versiegelungs- und Schutzüberzugsmassen, Kronen- und Brückenmaterialien, Prothesenmaterialien, sowie Massen zur Herstellung künstlicher Zähne) bekannt, der neben polymerisierbarem Acrylat beziehungsweise Methacrylat als anorganischen Füllstoff mikrofeines (hochdisperses) Siliciumdioxid mit einer Teilchengröße von etwa 10-400 mµ enthält. Das polymerisierbare Monomer besteht dabei aus Bis-GMA oder einem anderen Derivat des Bisphenol A oder einem Reaktionsprodukt aus Hydroxyalkylmethacrylaten und Diisocyanaten, gegebenenfalls zusammen mit monomeren kurzkettigen Methacrylaten und/oder Diacrylaten beziehungsweise Dimethacrylaten. Die aus den den mikrofeinen Füllstoff enthaltenden Werkstoff hergestellten Zahnfüllungen und dergleichen zeichnen sich durch ihre Hochglanzpolierbarkeit aus.

Aus der deutschen Patentschrift 24 05 578 ist es bekannt, in einem zu hochglanzpolierbaren Produkten zu verarbeitenden Dentalwerkstoff neben Estern der Methacrylsäure als anorganischen Füllstoff eine Mischung aus Kieselsäure mit einer maximalen Teilchengröße von 0,07 Mikrometer und feinteiligem Glas, dessen Teilchengröße 5 Mikrometer nicht überschreiten sollte, zu verwenden. Als Methacrylsäureester werden darin Bis-GMA, 2,2-Bis-[p-(2-hydroxyäthoxy)-phenyl]-propandimethacrylat und Triäthylenglykoldimethacrylat genannt.

Ein sowohl konventionelle als auch mikrofeine anorganische Füllstoffe enthaltendes Dentalmaterial - dafür hat sich die Bezeichnung Hybrid-Composite eingebürgert - wird zum Beispiel auch in der internationalen Patentanmeldung WO81/02 254 beschrieben. Es enthält ein Füllstoff-Gemisch aus hydrophobem Siliciumdioxid mit einem Durchmesser von 0,01-0,04 Mikrometer und Glas, zum Beispiel röntgenopakes Barium- oder Strontium-haltiges Glas, mit einem Durchmesser von 2-30 Mikrometer. Als polymerisierbare Monomere dienen Bis-GMA oder äthoxyliertes Bisphenol A-dimethacrylat und Triäthylenglykoldimethacrylat. Das Material wird als Zahnfüllungsmaterial und zum Verblenden von zum Beispiel gegossenen Gold-Kronen verwendet.

Aus der europäischen Patentanmeldung 0 156 105, EP-A-0 156 105, ist ein polymerisierbares dentales Füllungsmaterial auf (Meth)Acrylat-Basis bekannt, das 60 - 99 Gewichts-% eines anorganischen Füllstoff-Gemisches aus 5 - 20 Gewichts-% eines röntgenopaken Füllstoffs mit einer Korngrößenverteilung zwischen 0,5 und 40 Mikrometer, 20 - 40 Gewichts-% eines röntgenopaken Füllstoffs mit einer Korngrößenverteilung zwischen 0,2 und 15 Mikrometer und 45 - 75 Gewichts-% eines Mikrofüllstoffs mit einer Korngrößenverteilung zwischen 5 und 150 Nanometer enthält. Die Füllstoffe können silanisiert sein, zum Beispiel mit Methacrylpropyltrihydroxysilan; als Mikrofüllstoff wird Siliciumdioxid bevorzugt. Das Füllungsmaterial läßt sich unter Lichteinfluß gut aushärten.

Die in der europäischen Patentanmeldung 0 382 033, EP-A-0 382 033, beschriebenen photopolymerisierbaren Dentalmassen enthalten neben monomeren (Meth)Acrylsäureestern als anorganische Füllstoffe 5 - 80 Gewichts-% eines silanisierten Glas- oder Glaskeramik-Pulvers mit einer mittleren Teilchengröße zwischen 0,1 und 10 Mikrometer und 2 - 10 Gewichts-% eines Mikrofüllers.

Die deutschen Patentschriften 37 08 618 und 38 26 233 betreffen Kunststoff-Zahnersatzteile mit einem abrasionsfesten, hochglanzpolierbaren Mantel aus 10-90 Gewichts-% hochdisperses Siliciumdioxid mit einer Teilchengröße von 0,01-0,4 Mikrometer enthaltendem Kunststoff. Der Mantel umhüllt einen eine hohe Biegefestigkeit und einen hohen Biegemodul aufweisenden Kern, der 30-90 Gewichts-% eines anorganischen Füllstoff-Gemisches aus 60-100 Gewichts-% Siliciumdioxid, Lithiumaluminiumsilicat-Glas und/oder Strontiumaluminiumsilicat-Glas mit einer mittleren Teilchengröße von 0,7 - 5 Mikrometer oder Bariumaluminiumsilicat-Glas mit einer mittleren Teilchengröße von 0,7 - 10 Mikrometer und 0 - 40 Gewichts-% hochdispersem Siliciumdioxid mit einer mittleren Teilchengröße von 0,01 - 0,4 Mikrometer enthält. Der Kunststoff von Kern und Mantel ist vorzugsweise ein Polymer aus Bis-GMA, äthoxyliertem Bisphenol A-diacrylat beziehungsweise -dimethacrylat, Triäthylenglykoldimethacrylat, Dodecandioldimethacrylat, Diurethandimethacrylat aus 2-Hydroxyäthylmethacrylat und 2,2,4-Trimethylhexamethylen-diisocyanat, Bis-(acryloyloxymethyl)-tricyclo-[5.2.1.0^{2,6}]decan und/oder Bis-(methacryloyloxymethyl)-tricyclo[5.2.1.0^{2,6}]decan. Die Zahnersatzteile eignen sich für die Versorgung mit Kronen, Brücken, Inlays und dergleichen.

Dentale Befestigungszemente werden benutzt, um Inlays, Onlays, Kronen, Brücken, auch sogenannte Klebe- oder Adhäsivbrücken (Maryland-Brücken), Verblendschalen und dergleichen mit der Zahnsubstanz zu verbinden. Neben infolge von Abbindungsvorgängen härtenden Zementen, wie zum Beispiel den Zinkoxid-Phosphat-Zementen, sind zunehmend auch solche in Gebrauch, die durch Polymerisation aushärten. Die polymerisierbaren Befestigungszemente enthalten als Monomere üblicherweise Ester der Acrylsäure beziehungsweise Methacrylsäure, meist einen feinteiligen anorganischen Füllstoff und daneben die Polymerisation auslösende Katalysatoren.

Aus EP-B1 0 064 834 ist ein Klebstoff zum Ankleben eines Gegenstandes an einen Zahn bekannt, der ein Bindemittelhart, verdünnendes Monomer, einen anorganischen Füllstoff in einer Menge von mindestens 20 Gewichts-Prozent und einen Photoinitiator zur Auslösung der Polymerisation bei Bestrahlung mit sichtbarem Licht enthält. Als Photoinitiator wird ein Gemisch aus einem α-Diketon, ausgewählt aus zum Beispiel Campherchinon, Benzil, Biacetyl, 9,10-Phenanthrenchinon und Naphthochinon, und einem Amin, besonders einem Dialkanol- oder Trialkanolamin, eingesetzt. Als Füllstoffe werden anorganische Gläser, wie zum Beispiel Bariumaluminiumsilicatglas und Lithiumaluminiumsilicatglas, bevorzugt.

Nach der deutschen Patentschritt 34 41 564 lassen sich die Metallflächen von Adhäsivbrücken fest, dicht und spaltfrei mit dem Zahnschmelz verbinden, wenn der dafür benutzte Klebstoff neben Methacrylsäureestern und anorganischem Füllstoff aus silanisiertem Siliciumdioxid mit einer Teilchengröße bis zu 0,04 Mikrometer sowohl einen Katalysator für die chemische Kaltpolymerisation (Autopolymerisation) als auch einen Katalysator für die Photopolymerisation enthält.

Aus Schweiz Monatsschr Zahnmed. Vol. 99, 4/1989, ist ein niedrigviskoser mikrogefüllter Komposit-Zement bekannt, der durch zweistufige Photopolymerisation gehärtet wird und zunächst gelb gefärbt ist und erst durch die Endhärtung seine definitive Farbe erhält. Dieser Zement enthält zwei Initiator-Systeme mit hohem Campherchinon-Anteil für die Photopolymerisation, deren Absorptionsmaxima bei verschiedenen Wellenlängen des sichtbaren Lichtes liegen. Das Anhärten erfolgt mit Licht einer Wellenlänge, die größer als 470 nm ist, das Endhärten mit Licht der Wellenlänge von rund 470 nm. Durch die zunächst von der Zahnfarbe abweichende Farbe des Zements und seine nach dem Anhärten marzipanähnliche Konsistenz läßt sich der Zement gut bearbeiten und ein Überschuß an Zement, ohne daß die Zahnsubstanz beschädigt wird, rasch und sicher entfernen. Dieser Zement eignet sich nicht für Restaurationen mit lichtunzugänglichen Bezirken.

Der Erfindung liegt die Aufgabe zugrunde, ein photopolymerisierbares Dentalmaterial der eingangs charakterisierten Art zu finden, aus dem sich durch Polymerisation Produkte herstellen lassen, deren Charakteristika Röntgenopazität, Abrasionsfestigkeit, Hochglanzpolierbarkeit und hohe mechanische Festigkeitswerte, speziell hohe Biegefestigkeits- und Biegemodulwerte, sind. Das Dentalmaterial soll als Füllungsmaterial für den Front- und Seitenzahnbereich, zur Herstellung von Inlays und als Befestigungszement eingesetzt werden können.

Das die Lösung der Aufgabe darstellende Dentalmaterial ist erfindungsgemäß dadurch gekennzeichnet, daß es im wesentlichen aus 10-60 Gewichts-% Bis-[4-(2-hydroxy-3-methacryloyloxypropoxy)-phenyl]-dimethylmethan, Triäthylenglykoldimethacrylat und/oder dem Diurethandimethacrylat aus 2,2,4-Trimethylhexamethylendiisocyanat und 2-Hydroxyäthylmethacrylat, 37-87 Gewichts-% eines Füllstoff-Gemisches aus 80-90 Gewichts-% Bariumaluminiumsilicat-Glas mit einer mittleren Teilchengröße von 0,7 Mikrometer und 10-20 Gewichts-% mikrofeinem Siliciumdioxid mit einer mittleren Teilchengröße von 0,04-0,06 Mikrometer, 0,02-2 Gewichts-% α-Diketon und 0,1-1 Gewichts-% Amin besteht.

Das anorganische Füllstoff-Gemisch wird vorzugsweise in zum Beispiel durch Behandlung mit 3-Methacryloyloxypropyltrimethoxysilan silanisierter Form eingesetzt.

Geeignete α-Diketon/Amin-Systeme, die bei der Bestrahlung mit sichtbarem Licht einer Wellenlänge von 320-500 Nanometer die Polymerisation der Monomeren auslösen, sind zum Beispiel aus GB-B-1 1 408 265 bekannt. Als α-Diketon hat sich Campherchinon besonders bewährt. Soll das Material als zunächst gefärbter, nach Bestrahlung mit sichtbarem Licht jedoch zahnfarbener Befestigungszement zur Verfügung stehen, so wird als α-Diketon ein Gemisch aus 0,01-1 Gewichts-% Campherchinon und 0,01-1 Gewichts-% 9,10-Phenanthrenchinon eingesetzt. Bevorzugt werden dabei 0,02-0,05 Gewichts-% Campherchinon, 0,02 - 0,05 Gewichts-% 9,10-Phenanthrenchinon und 0,3-0,5 Gewichts-% Amin.

Als Amine haben sich N,N-Bis-(2-hydroxyäthyl)-p-toluidin und Ester der 4-Dimethylaminobenzoesäure, wie der Äthyl- und Butoxyäthylester, besonders bewährt.

Es hat sich als günstig erwiesen, dem Dentalmaterial als weiteren photoaktiven Bestandteil zusätzlich eine Benzoylverbindung, zum Beispiel ein Benzilacetal, vorzugsweise in einer Menge von 0,02-0,1 Gewichts-%, zuzufügen.

Dem Dentalmaterial können außerdem noch Farbpigmente, Antioxidationsmittel, UV-Stabilisatoren und andere übliche Zusatzstoffe beigefügt werden.

Besonders bewährt hat sich das Dentalmaterial als Befestigungszement, wenn es neben dem Photoinitiator-System auch einen Katalysator für die Autopolymerisation enthält. Mit einem solchen Zement läßt sich, da sein Aushärten sowohl chemisch als auch photochemisch erfolgt, Zahnersatz auch in lichtunzugänglichen Bereichen, in denen keine Photopolymerisation möglich ist, fest mit der Zahnsubstanz verbinden.

Für den Zweck der zusätzlichen Autopolymerisation enthält das Dentalmaterial vorzugsweise ein organisches Peroxid, zum Beispiel Diacetylperoxid, Dibenzoylperoxid oder Di-tert.-butylperoxid, das zusammen mit dem Amin ein Redox-System bildet. Redox-Systeme dieser Art stellen bekannte Katalysatoren für die Autopolymerisation von Dentalmaterialien auf Acrylat- beziehungsweise Methacrylat-Basis dar.

Das erfindungsgemäße Dentalmaterial zeichnet sich durch eine hohe Haftung an dem Zahngewebe und eine gute Bearbeitbarkeit und Modellierfähigkeit, die daraus hergestellten Produkte durch Röntgenopazität, Abrasionsfestigkeit, Hochglanzpolierbarkeit und gute mechanische Eigenschaften - besonders charakteristisch sind dabei die hohe Biegefestigkeit (120 MPa) und der hohe Biegemodul (8000-120000 MPa) - aus.

Für den Gebrauch hat es sich bewährt, das Dentalmaterial, wenn es Methacrylsäureester, anorganischen Füllstoff, Photoinitiator-System und gegebenenfalls gewünschte Zusatzstoffe enthält, als lagerfähiges Einkomponenten-Material in Pasten-Form zur Verfügung zu stellen.

Stellt das Dentalmaterial einen auch durch Autopolymerisation zu härtenden Befestigungszement dar, so liegt dieser vorzugsweise als pastenförmiges Zweikomponenten-Material vor, wobei zweckmäßigerweise die Zusammensetzung der einen Paste der des Einkomponenten-Materials entspricht und die andere Paste neben Methacrylsäureester und anorganischem Füllstoff den Katalysator für die Autopolymerisation, das Peroxid, enthält.

Der Befestigungszement gemäß der Erfindung eignet sich zur Befestigung jeglicher Art von aus Keramik, Glaskeramik oder Composite-Material gefertigtem Zahnersatz und von Klebebrücken an der Zahnsubstanz.

Soll zum Beispiel mit Hilfe des Campherchinon und 9,10- Phenanthrenchinon enthaltenden Befestigungszements eine Zahnkavität mit einem aus einem Composite-Material gefertigten Inlay gefüllt werden, so wird zunächst der Befestigungszement auf die Wände der Kavität und auf das Inlay, soweit es in die Kavität reicht, aufgetragen. Dann wird das Inlay in die Kavität gedrückt. Ein Überschuß an Befestigungszement, der sich durch seine gelbe Farbe deutlich sichtbar von der Zahnsubstanz abhebt und eine weiche Konsistenz besitzt, wird mit einem geeigneten Instrument abgenommen, ohne daß dabei die Zahnsubstanz beschädigt und in der Spalte zwischen Kavität und Inlay vorhandener Befestigungszement entfernt wird. Anschließend wird der Befestigungszement mit einem Lichtgerät, wie es zum Beispiel für das Aushärten von photopolymerisierbarem Zahnfüllungsmaterial bekannt ist, so lange bestrahlt, bis die gelbe Färbung verschwunden und damit der Befestigungszement vollständig ausgehärtet ist.

Zur näheren Erläuterung werden in den folgenden Beispielen Ausführungsformen des Dentalmaterials gemäß der Erfindung beschrieben.

### Beispiel 1

Durch Photopolymerisation auszuhärtendes Zahnfüllungs- und Inlaymaterial

| | Gewichts-% |
|---|---|
| Bis-GMA | 14 |
| Triäthylenglykoldimethacrylat | 7 |
| Bariumaluminiumsilicat-Glas¹, mittlere Teilchengröße 0,7 Mikrometer | 69,7 |
| Siliciumdioxid¹, mittlere Teilchengröße 0,04-0,06 Mikrometer | 8 |
| Campherchinon | 0,4 |
| Benzildimethylacetal², 10 %ige Lösung in Triäthylenglykoldimethacrylat | 0,6 |
| 4-Dimethylaminobenzoesäurebutoxyäthylester | 0,3 |

### Beispiel 2

Durch Photopolymerisation auszuhärtender Befestigungszement in Form einer Paste

| | Gewichts-% |
|---|---|
| Bis-GMA | 30,00 |
| Triäthylenglykoldimethacrylat | 16,58 |
| Bariumaluminiumsilicat-Glas¹, mittlere Teilchengröße 0,7 Mikrometer | 40,80 |
| Siliciumdioxid¹, mittlere Teilchengröße 0,04-0,06 Mikrometer | 10,20 |
| Campherchinon, 10 %ige Lösung in Triäthylenglykoldimethacrylat | 1,02 |
| Benzildimethylacetal², 10 %ige Lösung in Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

### Beispiel 3

Durch Photopolymerisation auszuhärtender Befestigungszement in Form einer Paste

| | Gewichts-% |
|---|---|
| Bis-GMA | 13,51 |
| Triäthylenglykoldimethacrylat | 7,28 |
| Bariumaluminiumsilicatglas¹, mittlere Teilchengröße 0,7 Mikrometer | 66,00 |
| Siliciumdioxid¹, mittlere Teilchengröße 0,04-0,06 Mikrometer | 10,79 |
| Campherchinon, 10 %ige Lösung in Triäthylenglykoldimethacrylat | 1,00 |
| 9,10-Phenanthrenchinon | 0,02 |
| Benzildimethylacetal², 10 %ige Lösung in Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

| | |
|---|---|
| ¹silanisiert mit 3-Methacryloyloxypropyltrimethoxysilan | |
| ² 1,2-Diphenyl-2,2-dimethoxyäthanon | |

### Beispiel 4

Durch Photopolymerisation und Autopolymerisation auszuhärtender Befestigungszement in Form zweier Pasten

| Paste A | Gewichts-% |
|---|---|
| Bis-GMA | 13,51 |
| Triäthylenglykoldimethacrylat | 7,28 |
| Bariumaluminiumsilicatglas¹, mittlere Teilchengröße 0,7 Mikrometer | 66,00 |
| Siliciumdioxid¹, mittlere Teilchengröße 0,04-0,06 Mikrometer | 10,79 |
| Campherchinon, 10 %ige Lösung in Triäthylenglykoldimethacrylat | 1,00 |
| 9,10-Phenanthrenchinon | 0,02 |
| Benzildimethylacetal², 10 %ige Lösung in Triäthylenglykoldimethacrylat | 0,60 |
| N,N-Bis-(2-hydroxyäthyl)-p-toluidin | 0,40 |
| 2-Hydroxy-4-n-octyloxybenzophenon (UV-Stabilisator) | 0,40 |

| | |
|---|---|
| ¹silanisiert mit 3-Methacryloyloxypropyltrimethoxysilan | |
| ² 1,2-Diphenyl-2,2-dimethoxyäthanon | |

| Paste B | Gewichts-% |
|---|---|
| Diurethandimethacrylat aus 1 mol 2,2,4-Trimethylhexa methylendiisocyanat und 2 mol 2-Hydroxyäthylmethacrylat 15,33 Triäthylenglykoldimethacrylat | 6,58 |
| Bariumaluminiumsilicatglas¹, mittlere Teilchengröße 0,7 Mikrometer | 68,12 |
| Siliciumdioxid,¹ mittlere Teilchengröße 0,04-0,06 Mikrometer | 9,53 |
| Dibenzoylperoxid | 0,44 |

| | |
|---|---|
| ¹silanisiert mit 3-Methacryloyloxypropyltrimethoxysilan | |
| ² 1,2-Diphenyl-2,2-dimethoxyäthanon | |

Vor Gebrauch werden die Pasten A und B im Verhältnis 1:1 miteinander vermischt.

## Patentansprüche

1. Polymerisierbares Dentalmaterial, enthaltend monomere Dimethacrylate, ein anorganisches Füllstoff-Gemisch aus Bariumaluminiumsilicat-Glas und mikrofeinem Siliciumdioxid und ein α-Diketon/Amin-System für die Photopolymerisation, dadurch gekennzeichnet, daß es im wesentlichen aus 10-60 Gewichts-% Bis-[4-(2-hydroxy-3-methacryloyloxypropoxy)-phenyl]-dimethylmethan, Triäthylenglykoldimethacrylat und/oder dem Diurethandimethacrylat aus 2,2,4-Trimethylhexamethylendiisocyanat und 2-Hydroxyäthylmethacrylat, 37-87 Gewichts-% eines Füllstoff-Gemisches aus 80-90 Gewichts-% Bariumaluminiumsilicatglas mit einer mittleren Teilchengröße von 0,7 Mikrometer und 10-20 Gewichts-% mikrofeinem Siliciumdioxid mit einer mittleren Teilchengröße von 0,04-0,06 Mikrometer, 0,02-2 Gewichts-% α-Diketon und 0,1-1 Gewichts-% Amin besteht.

2. Dentalmaterial nach Anspruch 1, dadurch gekennzeichnet, daß das anorganische Füllstoff-Gemisch silanisiert ist.

3. Dentalmaterial nach Anspruch 1 oder 2, dadurch gekennzeichnet, daß es zusätzlich 0,02-0,1 Gewichts-% eines Benzilacetals enthält.

4. Dentalmaterial nach einem der Ansprüche 1 bis 3, dadurch gekennzeichnet, daß es als α-Diketon Campherchinon enthält.

5. Dentalmaterial nach einem der Ansprüche 1 bis 3, dadurch gekennzeichnet, daß es als α-Diketon ein Gemisch aus 0,01-1 Gewichts-% Campherchinon und 0,01-1 Gewichts-% 9,10-Phenanthrenchinon enthält.

6. Dentalmaterial nach Anspruch 5, dadurch gekennzeichnet, daß es 0,02-0,05 Gewichts-% Campherchinon, 0,02-0,05 Gewichts-% 9,10-Phenanthrenchinon und 0,3-0,5 Gewichts-% Amin enthält.

7. Dentalmaterial nach Anspruch 6, dadurch gekennzeichnet, daß es zusätzlich 0,2-0,5 Gewichts-% eines organischen Peroxids enthält.

8. Verwendung des Dentalmaterials nach einem der Ansprüche 1 bis 4 zur Herstellung von Zahnfüllungen und Inlays.

9. Verwendung des Dentalmaterials nach einem der Ansprüche 1 bis 7 als Befestigungszement.

## Claims

1. Polymerisable dental material, containing monomeric dimethacrylate, an inorganic filler mixture of barium aluminium silicate glass and microfine silicon dioxide and an α-diketone/amine system for photopolymerisation, characterised in that it consists substantially of 10-60% by weight bis-[4-(2-hydroxy-3-methacryloylpropoxy)-phenyl]-dimethylmethane, triethylene glycol dimethacrylate and/or the diurethanedimethacrylate of 2,2,4-trimethylhexamethylene diisocyanate and 2-hydroxyethylmethacrylate, 37-87% by weight of a filler mixture of 80-90% by weight barium aluminium silicate glass with a mean particle size of 0.7 micrometres and 10-20% by weight microfine silicon dioxide with a mean particle size of 0.04- 0.06 micrometres, 0.02-2% by weight α-diketone and 0.1-1% by weight amine.

2. Dental material according to claim 1, characterised in that the inorganic filler mixture is silanised.

3. Dental material according to claim 1 or 2, characterised in that it additionally contains 0.02-0.1% by weight of a benzil acetal.

4. Dental material according to one of claims 1 to 3, characterised in that it contains camphor quinone as α-diketone.

5. Dental material according to one of claims 1 to 3, characterised in that it contains a mixture of 0.01-1% by weight camphor quinone and 0.01-1% by weight 9,10-phenanthrene quinone.

6. Dental material according to claim 5, characterised in that it contains 0.02-0.05% by weight camphor quinone, 0.02-0.05% by weight 9,10-phenanthrene quinone and 0.3-0.5% by weight amine.

7. Dental material according to claim 6, characterised in that it additionally contains 0.2-0.5% by weight of an organic peroxide.

8. Use of the dental material according to one of claims 1 to 4 for the manufacture of dental fillings and inlays.

9. Use of the dental material according to one of claims 1 to 7 as securing cement.

## Revendications

1. Matière dentaire polymérisable contenant des diméthacrylates monomères, un mélange de masses de remplissage inorganique constitué de verre de silicate de baryum-aluminium et de dioxyde de silicium à particules microfines et un système α-dicétone/amine pour la photopolymérisation, caractérisée en ce qu'elle est composée essentiellement par 10-60 % en masse de bis-[4-(2-hydroxy-3-méthacryloyloxypropoxy)-phényl]-diméthylméthane, par du diméthacrylate de triéthylèneglycol et/ou par du diméthacrylate de diuréthanne constitué de diisocyanate de 2,2,4-triméthylhexaméthylène et de méthacrylate de 2-hydroxyéthyle, par 37-87 % en masse d'un mélange de masse de remplissage constitué de 80-90% en masse de verre de silicate de baryum-aluminium ayant une dimension moyenne de particules de 0,7 µm et de 10-20 % en masse de dioxyde de silicium à particules microfines ayant une dimension moyenne de particules de 0,04 à 0,06 µm, par 0,02-2 % en masse d'α-dicétone et par 0,1-1 % en masse d'amine.

2. Matière dentaire selon la revendication 1, caractérisée en ce que le mélange de masses de remplissage est silané.

3. Matière dentaire selon la revendication 1 ou 2, caractérisée en ce qu'elle contient additionnellement de 0,02 à 0,1 % en masse d'un benzilacétal.

4. Matière dentaire selon l'une quelconque des revendications 1 à 3, caractérisée en ce qu'elle contient de la camphoquinone en tant qu'α-dicétone.

5. Matière dentaire selon l'une quelconque des revendications 1 à 3, caractérisée en ce qu'elle contient un mélange constitué de 0,01-1 % en masse de camphoquinone et de 0,01-1 % en masse de 9,10-phénanthrènequinone en tant qu'α-dicétone.

6. Matière dentaire selon la revendication 5, caractérisée en ce qu'elle contient de 0,02 à 0,05 % en masse de camphoquinone, de 0,02 à 0,05 % en masse de phénanthrènequinone et de 0,3 à 0,5 % en masse d'amine.

7. Matière dentaire selon la revendication 6, caractérisée en ce qu'elle contient additionnellement de 0,2 à 0,5 % en masse d'un peroxyde organique.

8. Utilisation de la matière dentaire selon l'une quelconque des revendications 1 à 4 en vue de préparer des obturations dentaires et des inlays.

9. Utilisation de la matière dentaire selon l'une quelconque des revendications 1 à 7 en tant que ciment de fixation.
